# EUROPEAN PATENT SPECIFICATION

(11) **EP 1 344 774 B1**
(45) Date of publication and mention of the grant of the patent: **07.12.2005**
(21) Application number: 03005826.7
(22) Date of filing: 14.03.2003
(51) Int. Cl.: C07D 231/38

(54) **Synthesizing methods of dicyanomethylidene compound and heterocyclic compound**
Verfahren zur Herstellung von dicyanomethyliden und heterozyklische Verbindungen
Méthodes de synthèse de derivés de 3-dicyanomethylidène et de dérivés hétérocycliques

(30) Priority: 15.03.2002 JP 2002072531
(43) Date of publication of application: 17.09.2003
(73) Proprietor: FUJI PHOTO FILM CO., LTD., Kanagawa (JP)
(72) Inventor: Matsuoka, Koshin, Minami-Ashigara-shi, Kanagawa (JP)
(74) Representative: Grünecker, Kinkeldey, Stockmair & Schwanhäusser Anwaltssozietät

(56) References cited:
- EP-A- 0 484 814
- US-A- 4 032 322
- SOUTHWICK, PHILIP L.; DHAWAN BALRAM: "Preparation of 4,6-Diaminopyrazolo[3,4-d]pyrimidines with Variation in Substitution at the 1- and 3-Positions" J.HET.CHEM, vol. 12, 1975, pages 1199-1205, XP002248196

## Description

The present invention relates to synthesizing methods of a dicyanomethylidene compound and a heterocyclic compound.

### BACKGROUND OF THE INVENTION

Aminopyrazoles represented by formula (V) are useful as the intermediates and starting materials of functional compounds, such as dyes and medicines. Aminopyrazoles are also useful since they are intermediates for synthesizing other heterocyclic compounds as disclosed in U.S. Patent 5,294,612.

General synthesis methods of these aminopyrazoles are described, e.g., in Hiroshi Yamanaka et al., Hetero-kan Kagobutsu noKagaku (Chemistry of Heterocyclic Compounds, Chap. 5, Kodansha Scientific (1988), J.A. Joule and K. Mills, Heterocyclic Chemistry, 4th Ed., Chap. 22, Blackwell Science (2000), A.R. Katritzky and A.F. Pozharskii, Handbook of Heterocyclic Chemistry, 2nd Ed., Chap. 4.3.2.3, Pergamon (2000), and A.R. Katritzky and C.W. Rees, Comprehensive Heterocyclic Chemistry. Vol. 5, Chap. 4.04.3, Pergamon (1984). Aminopyrazoles can be easily synthesized by the reaction of β-ketonitrile compounds with hydrazines.

Specifically, for example, it is reported in J. Org. Chem., 52, 25, 1987, 5538 that 5-amino-3-t-butylpyrazole in the yield of 82% can be easily synthesized by heating pivaloylacetonitrile and hydrazine hydrate in an ethanol solvent for one hour.

On the other hand, it is reported in Bull. Chim. Soc. Fr., 1965, 3323 that 3-substituted-5-amino-4-cyanopyrazole cannot be obtained by reacting acylmalononitrile derivative represented by formula (I) directly with hydrazines in the same manner as the above synthesizing route. In practice, 5-amino-4-cyano-3-pivaloylpyrazole cannot be obtained even when pivaloylmalononitrile and hydrazine in an isopropanol solvent at 80°C. US 4,032,322 discloses a class of herbicidal compounds and methods for the synthesis thereof. EP 484 814 A1 discloses a magenta dye down element.

Synthesizing methods of 3-substituted-5-amino-4-cyanopyrazoles which have been reported heretofore are as follows.
(a) Methods of converting acylmalononitrile into enol ether under a basic condition by dimethylsulfuric acid or diazomethane (a compound represented by formula (II), wherein X represents -OCH₃ or -OC₂H₅), and then reacting the enol ether with hydrazine (e.g., J. Heterocyclic Chem., 12, 1199 (1975), Bull. Chim. Soc. Fr., 3323 (1965), J. Chem. Soc., Perkin Trans., 1, 1545 (1996), and U.S. Patent 5,294,612),
(b) Methods of converting acylmalononitrile into a 3-chloro-2-cyanoacrylonitrile derivative (a compound represented by formula (II), wherein X represents -Cl) by the reaction with phosphorus pentachloride, and reacting the 3-chloro-2-cyanoacrylonitrile derivative with hydrazine (e.g., U.S. Patent 5,079,213, EP-A-0492444),
(c) Methods of synthesizing a 3-amino-2-cyanoacrylonitrile derivative and reacting the compound with hydrazine (e.g., J. Heterocyclic Chem., 29, 1067 (1992)),
(d) Methods of synthesizing a 3-alkoxy-2-cyanoacrylonitrile derivative (a compound represented by formula (II), wherein X represents an alkoxyl group) from orthoester and malononitrile, and reacting the 3-alkoxy-2-cyanoacrylonitrile derivative with hydrazine (e.g., J. Org. Chem., 21, 1240 (1956)), and
(e) Methods of preparing a methylthioamidium salt from a thioamide derivative and dimethylsulfuric acid, reacting the methylthioamidium salt with malononitrile, and then reacting the compound with hydrazine, or synthesizing a 3-methylthio-2-cyanoacrylonitrile derivative (a compound represented by formula (II), wherein X represents -SCH₃) from thioamide or dithiocarboxylate and tetracyanoethylene oxide, and then reacting the compound with hydrazine (J. Heterocyclic Chem. , 27, 647 (1990)).

These synthesizing methods have problems as described below.

In method (a), toxic dimethylsulfuric acid or diazomethane which is not easy to handle must be used as the alkylating agent.

In method (b), since a large quantity of phosphorus pentachloride is used for chlorination, charging for reaction and post treatment are not easy. For example, in U.S. Patent 5,079,213, although the yield is not described, 57 g of a compound represented by formula (II) (wherein R²¹ represents t-Bu) and 86 g of phosphorus pentachloride are reacted in 500 ml of methylene chloride. That is, about 1.1 equivalent amount of phosphorus pentachloride is used, and not all the chlorine atoms on the phosphorus pentachloride are used for chlorination. Further, the use of a halide-based solvent is not preferred, since it maleficently affects environment.

In method (c), the syntheses of matrices are limited to those which are easily synthesized. In many cases, as described in DE-A-2434922, Liebigs Ann. Chem., 884 (1982), and J. Heterocyclic Chem., 29, 1067 (1992), the syntheses require several processes.

Inmethod (d), limited orthoesters of starting materials are easily available, but when a desired orthoester is not available, the step of synthesizing the orthoester is necessary, as a result, the number of entire processes is increased.

In method (e), thioamide derivative and dithiocarboxylate of starting materials are also not easily available, thus the number of processes increases the same as the above (d). Further, tetracyanoethylene oxide is not an inexpensive material.

As described above, a simple synthesizing method of a compound represented by formula (V) has been desired.

### SUMMARY OF THE INVENTION

An object of the present invention is to provide a method of synthesizing a heterocyclic compound represented by formula (V) in short steps and efficiently without using toxic compounds, compounds which are not easy to handle or expensive starting materials, and another object is to provide a method of synthesizing a dicyanomethylidene compound represented by formula (II) which is preferably used as the starting material of the heterocyclic compound represented by formula (V).

The above objects of the present invention have been achieved by the synthesizing methods disclosed in claims 1 and 3. Preferred embodiments are defined in claims 2 and 4 to 8.
(1) A synthesizing method of a dicyanomethylidene compound represented by the following formula (II), which comprises: reacting a compound represented by the following formula (I) with a dehydrating agent; and then reacting the reaction product with a nucleophilic agent: 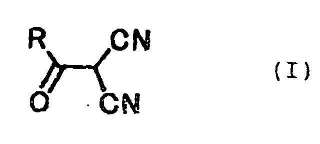 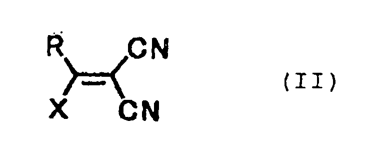 wherein R represents a hydrogen atom, a substituted or unsubstituted alkyl group, a substituted or unsubstituted aryl group, or a substituted or unsubstituted heterocyclic group; X represents a substituent that derives from the nucleophilic agent and is bonded via one of an oxygen atom, a nitrogen atom and a sulfur atom, and wherein the dehydrating agent is as defined in claim 1.
(2) A synthesizing method of a dicyanomethylidene compound represented by the following formula (II), which comprises: synthesizing a compound represented by the following formula (I) by reacting a compound represented by the following formula (III) with malononitrile; reacting the compound represented by the formula (I) with a dehydrating agent without taking out the compound represented by the formula (I); and then reacting the reaction product with a nucleophilic agent: 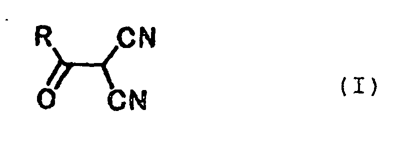 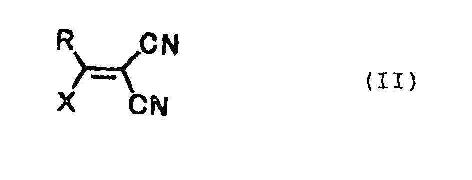 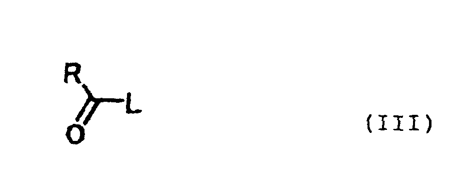 wherein R represents a hydrogen atom, a substituted or unsubstituted alkyl group, a substituted or unsubstituted aryl group, or a substituted or unsubstituted heterocyclic group; X represents a substituent that derives from the nucleophilic agent and is bonded via one of an oxygen atom, a nitrogen atom and a sulfur atom; L represents a releasing group, wherein the dehydrating agent is as defined in claim 3.
(3) A synthesizing method of a heterocyclic compound represented by the following formula (V), which comprises reacting the compound represented by formula (II) synthesized by the method described in (1) or (2) with a hydrazine compound represented by the following formula (IV):

   **H**_{**2**}**N-NHR'** (IV)

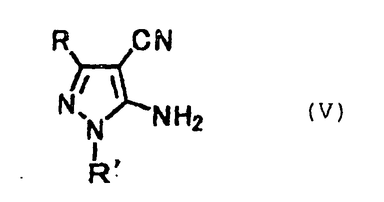

   wherein R' represents a hydrogen atom, a substituted or unsubstituted alkyl group, a substituted or unsubstituted aryl group, or a substituted or unsubstituted heterocyclic group.
(4) The synthesizing method described in the item (1), wherein the dehydrating agent is used in the molar ratio of from 1/10 to 50 to the compound represented by formula (I).
(5) The synthesizing method described in the item (1), wherein the nucleophilic agent is used in the molar ratio of from 1/10 to 50 to the compound represented by formula (I).
(6) The synthesizing method described in the item (2), wherein malononitrile is used in the molar ratio of from 1/10 to 10 to the compound represented by formula (III).
(7) The synthesizing method described in the item (3), wherein the hydrazine compound represented by formula (IV) is used in the molar ratio of from 1/10 to 10 to the compound represented by formula (II).

### DETAILED DESCRIPTION OF THE INVENTION

The present invention is described in detail below.

In the present invention, a dicyanomethylidene compound represented by the following formula (II) is synthesized by making a dehydrating agent and then a nucleophilic agent act on a compound represented by the following formula (I):

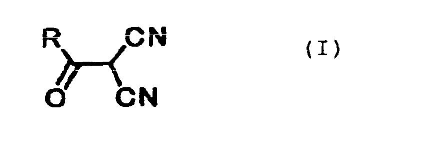

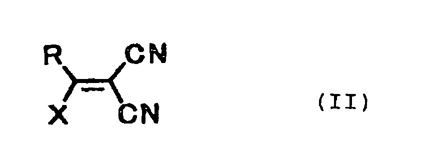

The dehydrating agent in the present invention means a compound which can take two hydrogen atoms and one oxygen atom from the same molecule or a plurality of molecules formally.

The dehydrating agents are selected from carboxylic acid halides (preferably carboxylic acid chloride, e.g., acetyl chloride, trichloroacetyl chloride, trifluoroacetyl chloride, pivaloyl chloride, benzoyl chloride, p-nitrobenzoyl chloride, oxalyl chloride), chlorocarbonic esters (e.g., methyl chlorocarbonate, ethyl chlorocarbonate, phenyl chlorocarbonate, p-nitrophenyl chlorocarbonate), sulfonic acid halides (preferably sulfonic acid chloride, e.g., methanesulfonic acid chloride, a benzenesulfonic acid chloride, p-toluenesulfonic acid chloride), carbodiimides (e.g. dicyclohexylcarbodiimide) isocyanates (e.g. methyl isocyanate, phenylisocyanate), carbamic acid chloride (dimethylcarbamic acid chloride, diethylcarbamic acid chloride), phosphorus oxychloride, cyanuric chloride, 1,1-carbonyl-bis-1H-imidazole, and imidazolinium salts (e.g., 2-chloro-1,3-dimethylimidazolinium chloride)

In making a dehydrating agent and then a nucleophilic agent act on a compound represented by formula (I), a base may be made to act on the compound represented by formula (I) before or at the same time with the dehydrating agent and the nucleophilic agent. The concentration of the compound represented by formula (I) at the time of reaction is preferably from 0.01 to 10 mol/liter, and more preferably from 0.1 to 5 mol/liter. It is preferred to use the dehydrating agent in the molar ratio of from 1/10 to 50 to the compound represented by formula (I).

The base may be an organic or inorganic base.

As the organic base, e.g., pyridines such as pyridine, collidine and lutidine, nitrogen-containing cyclic compounds such as DBU and DBN, amines such as diethylamine, triethylamine and N, N-dimethylaniline, and N,N,N',N'-tetramethylguanidine, are exemplified.

As the inorganic base, e.g., metallic hydroxides such as sodium hydroxide, potassium hydroxide, calcium hydroxide, magnesium hydroxide, lithium hydroxide, and vanadium hydroxide, metal alkoxides such as sodium methoxide, sodium ethoxide, sodium t-butoxide, and potassium t-butoxide, metal carbonates such as lithium carbonate, sodium carbonate, potassium carbonate, sodium hydrogencarbonate, and potassium hydrogencarbonate, hydrogencarbonate, metallic salts of carboxylic acid such as sodium acetate, potassium acetate, calcium acetate, and calcium oxalate, hydrogenatedmetals such as sodiumhydride and potassium hydride, metals such as sodium and potassium, and organic metallic compounds such as organic magnesium compounds and organic lithium compounds (e.g., methylmagnesium bromide, methylmagnesium chloride, ethylmagnesium bromide, ethylmagnesium chloride, phenylmagnesium bromide, phenylmagnesium chloride, t-butylmagnesium bromide, t-butylmagnesium chloride, n-butylmagnesium bromide, n-butylmagnesium chloride, methyl lithium, n-butyl lithium, t-butyl lithium, phenyl lithium), are exemplified.

The bases for use in the reaction of the present invention are preferably organic bases, and more preferably pyridines such as pyridine, collidine and lutidine, nitrogen-containing cyclic compounds such as DBU and DBN, and amines such as diethylamine, triethylamine and N,N-dimethylaniline are exemplified.

In the reaction of the present invention, bases can be used in the amount necessary for the reaction to progress to the highest yield, but in general the mol number of bases used is preferably from 0.2 to 5 times the mol number of the compound represented by formula (I), and more preferably from 0.5 to 3 times.

In the present invention, the nucleophilic agent is a compound capable of introducing substitution X into the position where X in the compound represented by formula (II) is substituted by the nucleophilic substitution reaction by acting on the compound represented by formula (I).

As the examples of X, substituted or unsubstituted alkoxyl, substituted or unsubstituted aryloxy, hydroperoxy, substituted or unsubstituted alkylhydroperoxy, substituted or unsubstituted arylhydroperoxy, amino, substituted or unsubstituted alkylamino, substituted or unsubstituted dialkylamino, substituted or unsubstituted arylamino, substituted or unsubstituted diarylamino, hydroxylamino, substituted or unsubstituted alkoxyamino, substituted or unsubstituted dialkoxyamino, substituted or unsubstituted hydrazino, thiol, substituted or unsubstituted alkylthio, substituted or unsubstituted arylthio, substituted or unsubstituted alkyldithio, and substituted or unsubstituted aryldithio are exemplified. As the preferred examples of X, alkoxyl, aryloxy, amino, mono- or dialkylamino, mono- or diarylamino, hydroxylamino, mono- or dialkoxyamino, hydrazino, thiol, alkylthio, and arylthio are exemplified. These substituents may have a substituent. It is preferred to use the nucleophilic agent in the molar ratio of from 1/10 to 50 to the compound represented by formula (I).

In the reaction of the invention, in synthesizing a dicyanomethylidene compound represented by formula (II) by making a dehydrating agent and then a nucleophilic agent act on a compound represented by formula (I), the reaction is preferably performed in the presence of a solvent. As the solvents, hydrocarbon-based solvents, e.g., toluene, xylene and hexane, halogen-based solvents, e.g., methylene chloride, sulfoxide-based solvents, e.g., dimethyl sulfoxide, amide-based solvents, e.g., dimethylformamide, ether-based solvents, e.g., tetrahydrofuran, dioxane and diglyme, alcohol-based solvents, e.g., methanol, ethanol and isopropanol, nitrile-based solvents, e.g., acetonitrile, ketone-based solvents, e.g., acetone and cyclohexanone, ester-based solvents, e.g., ethyl acetate, heterocyclic-based solvents, e.g., pyridine and piperidine, and water can be used. Two or more organic solvents may be used as mixture. The solvent may be a hydrous organic solvent or may be a two-phase series solvent of water-organic solvent. Further, an ionic liquid may be used as a reaction solvent as described in J. Chem. Tech. Biotechnol., 68, 351 (1997), or the reaction may be carried out by using a super-critical fluid as described in Science, 269, 1065 (1995).

Of the above solvents, hydrocarbon-based solvents, ether-based solvents, nitrile-based solvents, ester-based solvents and heterocyclic-based solvents are preferably used. Hydrocarbon-based solvents, e.g., toluene, nitrile-based solvents, e.g., acetonitrile, and ester-based solvents, e.g., ethyl acetate are more preferably used.

In the reaction of the present invention, it is preferred to carry out the reaction at -20°C to 100°C, and more preferably from -10°C to 80°C. The termination of the reaction can be confirmed by NMR, TLC, HPLC and other methods.

The time required for the reaction normally depends upon the reaction temperature, pH and the introducing time of bases, but the reaction time is generally about 0.1 to 24 hours.

It is preferred that the reaction is carried out for 0.1 to 24 hours when the temperature is from -10°C to 80°C.

The compounds in the present invention are described below.

In formulae (I) and (II), R represents a hydrogen atom, a substituted or unsubstituted alkyl group, a substituted or unsubstituted aryl group, or a substituted or unsubstituted heterocyclic group.

The alkyl group is a saturated or unsaturated, straight chain, branched, or cyclic alkyl group preferably having from 1 to 36 carbon atoms, more preferably from 1 to 30 carbon atoms, and still more preferably from 1 to 20 carbon atoms, which may have a substituent.

The aryl group is a monocyclic or condensed aryl group preferably having from 6 to 36 carbon atoms, and more preferably from 6 to 30 carbon atoms, which may have a substituent.

The heterocyclic group is preferably a monovalent group obtained by removing one hydrogen atom from a 5- or 6-membered, substituted or unsubstituted, aromatic or non-aromatic heterocyclic compound, and more preferably a 5- or 6-membered aromatic heterocyclic group having from 3 to 30 carbon atoms, which may have a substituent.

The examples of the substituents include, e.g., a halogen atom, an alkyl group (including a monocyclic or polycyclic cycloalkyl group), an alkenyl group (including a monocyclic or polycyclic cycloalkenyl group), an alkynyl group, an aryl group, a heterocyclic group, a cyano group, a hydroxyl group, a nitro group, a carboxyl group, an alkoxyl group, an aryloxy group, a silyloxy group, a heterocyclic oxy group, an acyloxy group, a carbamoyloxy group, an alkoxycarbonyloxy group, an aryloxycarbonyloxy group, an amino group (including an alkyl- or arylamino group), an acylamino group, an aminocarbonylamino group, an alkoxycarbonylamino group, an aryloxycarbonylamino group, a sulfamoylamino group, an alkyl- or arylsulfonylamino group, a mercapto group, an alkylthio group, an arylthio group, a heterocyclic thio group, a sulfamoyl group, a sulfo group, an alkyl- or arylsulfinyl group, an alkyl- or arylsulfonyl group, an acyl group, an aryloxycarbonyl group, analkoxycarbonyl group, a carbamoyl group, an aryl- or heterocyclic azo group, an imido group, a phosphino group, a phosphinyl group, a phosphinyloxy group, a phosphinylamino group, and a silyl group.

The more specific examples of the substituents include a halogen atom (e.g., fluorine, chlorine, bromine and iodine), an alkyl group [a straight chain, branched or cyclic, substituted or unsubstituted alkyl group including, e.g., an alkyl group (preferably an alkyl group having from 1 to 30 carbon atoms, e.g., methyl, ethyl, n-propyl, isopropyl, t-butyl, n-octyl, eicosyl, 2-chloroethyl, 2-cyanoethyl and 2-ethylhexyl), a cycloalkyl group (preferably a substituted or unsubstituted cycloalkyl group having from 3 to 30 carbon atoms, e.g., cyclohexyl, cyclopentyl and 4-n-dodecylcyclohexyl), a bicycloalkyl group (preferably a substituted or unsubstituted bicycloalkyl group having from 5 to 30 carbon atoms, i.e., a monovalent group obtained by removing one hydrogen atom from a bicycloalkane group having from 5 to 30 carbon atoms, e.g., bicyclo[1,2,2]heptan-2-yl and bicyclo[2,2,2]octan-3-yl), and tricyclo structure having more cyclic structures, and further, the alkyl groups in the substituents described below (e.g., the alkyl group in an alkynylthio group) are also included in the alkyl group of these concepts], an alkenyl group [a straight chain, branched or cyclic, substituted or unsubstituted alkenyl group including, e.g., an alkenyl group (preferably a substituted or unsubstituted alkenyl group having from 2 to 30 carbon atoms, e.g., vinyl allyl, prenyl, geranyl and oleyl), a cycloalkenyl group (preferably a substituted or unsubstituted cycloalkenyl group having from 3 to 30 carbon atoms, i.e., a monovalent group obtained by removing one hydrogen atom from a cycloalkene group having from 3 to 30 carbon atoms, e.g., 2-cyclopenten-1-yl and 2-cyclohexen-1-yl), a bicycloalkenyl group (a substituted or unsubstituted bicycloalkenyl group, preferably a substituted or unsubstituted bicycloalkenyl group having from 5 to 30 carbon atoms, i.e., a monovalent group obtained by removing one hydrogen atom from a bicycloalkene group having one double bond, e.g., bicyclo[2,2,1]hept-2-en-1-yl and bicyclo[2,2,2]oct-2-en-4-yl], an alkynyl group (preferably a substituted or unsubstituted alkynyl group having from 2 to 30 carbon atoms, e.g., ethynyl, propargyl and trimethylsilylethynyl), an aryl group (preferably a substituted or unsubstituted aryl group having from 6 to 30 carbon atoms, e.g., phenyl, p-tolyl, naphthyl, m-chlorophenyl, and o-hexadecanoylaminophenyl), a heterocyclic group (preferably a monovalent group obtained by removing one hydrogen atom from a 5- or 6-membered, substituted or unsubstituted, aromatic or non-aromatic heterocyclic compound, and more preferably a 5- or 6-membered aromatic heterocyclic group having from 3 to 30 carbon atoms, e.g., 2-furyl, 2-thienyl, 2-pyrimidinyl, and 2-benzothiazolyl), a cyano group, a hydroxyl group, a nitro group, a carboxyl group, an alkoxyl group (preferably a substituted or unsubstituted alkoxyl group having from 1 to 30 carbon atoms, e.g., methoxy, ethoxy, isopropoxy, t-butoxy, n-octyloxy, and 2-methoxyethoxy), an aryloxy group (preferably a substituted or unsubstituted aryloxy group having from 6 to 30 carbon atoms, e.g., phenoxy, 2-methylphenoxy, 4-t-butylphenoxy, 3-nitrophenoxy, and 2-tetradecanoylaminophenoxy), a silyloxy group (preferably a silyloxy group having from 3 to 20 carbon atoms, e.g., trimethylsilyloxy and t-butyldimethylsilyloxy), a heterocyclic oxy group (preferably a substituted or unsubstituted heterocyclic oxy group having from 2 to 30 carbon atoms, e.g., 2-phenyltetrazol-5-oxy and 2-tetrahydropyranyloxy), an acyloxy group (preferably formyloxy, a substituted or unsubstituted alkylcarbonyloxy group having from 2 to 30 carbon atoms, a substituted or unsubstituted arylcarbonyloxy group having from 7 to 30 carbon atoms, e.g., acetyloxy, pivaloyloxy, stearoyloxy, benzoyloxy, and p-methoxyphenylcarbonyloxy), a carbamoyloxy group (preferably a substituted or unsubstituted carbamoyloxy group having from 1 to 30 carbon atoms, e.g., N,N-dimethylcarbamoyloxy, N,N-diethylcarbamoyloxy, morpholinocarbonyloxy, N,N-di-n-octylaminocarbonyloxy, and N-n-octylcarbamoyloxy), an alkoxycarbonyloxy group (preferably a substituted or unsubstituted alkoxycarbonyloxy group having from 2 to 30 carbon atoms, e.g., methoxycarbonyloxy, ethoxycarbonyloxy, t-butoxycarbonyloxy, and n-octylcarbonyloxy), an aryloxycarbonyloxy group (preferably a substituted or unsubstituted aryloxycarbonyloxy group having from 7 to 30 carbon atoms, e.g., phenoxycarbonyloxy, p-methoxyphenoxycarbonyloxy, and p-(n-hexadecyloxy)phenoxycarbonyloxy), an amino group (preferably an amino group, a substituted or unsubstituted alkylamino group having from 1 to 30 carbon atoms, a substituted or unsubstituted arylamino group having from 6 to 30 carbon atoms, e.g., amino, methylamino, dimethylamino, anilino, N-methylanilino, and diphenylamino), an acylamino group (preferably formylamino, a substituted or unsubstituted alkylcarbonylamino group having from 2 to 30 carbon atoms, a substituted or unsubstituted arylcarbonylamino group having from 7 to 30 carbon atoms, e.g., acetylamino, pivaloylamino, lauroylamino, benzoylamino, and 3,4,5-tri-n-octyloxyphenylcarbonylamino), an aminocarbonylamino group (preferably a substituted or unsubstituted aminocarbonylamino group having from 1 to 30 carbon atoms, e.g., carbamoylamino, N,N-dimethylaminocarbonylamino, and N,N-diethylaminocarbonylamino, morpholinocarbonylamino), an alkoxycarbonylamino group (preferably a substituted or unsubstituted alkoxycarbonylamino group having from 2 to 30 carbon atoms, e.g., methoxycarbonylamino, ethoxycarbonylamino, t-butoxycarbonylamino, n-octadecyloxycarbonylamino, and N-methylmethoxycarbonylamino), an aryloxycarbonylamino group (preferably a substituted or unsubstituted aryloxycarbonylamino group having from 7 to 30 carbon atoms, e.g., phenoxycarbonylamino, p-chlorophenoxycarbonylamino, and m-(n-octyloxy)phenoxycarbonylamino), a sulfamoylamino group (preferably a substituted or unsubstituted sulfamoylamino group having from 0 to 30 carbon atoms, e.g., sulfamoylamino, N,N-dimethylaminosulfonylamino, and N-n-octylaminosulfonylamino), an alkyl- or arylsulfonylamino group (preferably a substituted or unsubstituted alkylsulfonylamino group having from 1 to 30 carbon atoms, a substituted or unsubstituted arylsulfonylamino group having from 6 to 30 carbon atoms, e.g., methylsulfonylamino, butylsulfonylamino, phenylsulfonylamino, 2,3,5-trichlorophenylsulfonylamino, and p-methylphenylsulfonylamino), a mercapto group, an alkylthio group (preferably a substituted or unsubstituted alkylthio group having from 1 to 30 carbon atoms, e.g., methylthio, ethylthio, and n-hexadecylthio), an arylthio group (preferably a substituted or unsubstituted arylthio group having from 6 to 30 carbon atoms, e.g., phenylthio, p-chlorophenylthio, and m-methoxyphenylthio), a heterocyclic thio group (preferably a substituted or unsubstituted heterocyclic thio group having from 2 to 30 carbon atoms, e.g., 2-benzothiazolylthio and 1-phenyltetrazol-5-ylthio), a sulfamoyl group (preferably a substituted or unsubstituted sulfamoyl group having from 0 to 30 carbon atoms, e.g., N-ethylsulfamoyl, N-(3-dodecyloxypropyl)sulfamoyl, N,N-dimethylsulfamoyl, N-acetylsulfamoyl, N-benzoylsulfamoyl, and N-(N'-phenylcarbamoyl)sulfamoyl), a sulfo group, an alkyl- or arylsulfinyl group (preferably a substituted or unsubstituted alkylsulfinyl group having from 1 to 30 carbon atoms, a substituted or unsubstituted arylsulfinyl group having from 6 to 30 carbon atoms, e.g., methylsulfinyl, ethylsulfinyl, phenylsulfinyl and p-methylphenylsulfinyl), an alkyl- or arylsulfonyl group (preferably a substituted or unsubstituted alkylsulfonyl group having from 1 to 30 carbon atoms, a substituted or unsubstituted arylsulfonyl group having from 6 to 30 carbon atoms, e.g., methylsulfonyl, ethylsulfonyl, phenylsulfonyl, and p-methylphenylsulfonyl), an acyl group (preferably formyl, a substituted or unsubstituted alkylcarbonyl group having from 2 to 30 carbon atoms, a substituted or unsubstituted arylcarbonyl group having from 7 to 30 carbon atoms, a substituted or unsubstituted heterocyclic carbonyl group having from 4 to 30 carbon atoms bonded to a carbonyl group via a carbon atom, e.g., acetyl, pivaloyl, 2-chloroacetyl, stearoyl, benzoyl, p-n-octyloxyphenylcarbonyl, 2-pyridylcarbonyl, and 2-furylcarbonyl), an aryloxycarbonyl group (preferably a substituted or unsubstituted aryloxycarbonyl group having from 7 to 30 carbon atoms, e.g., phenoxycarbonyl, o-chlorophenoxycarbonyl, m-nitrophenoxycarbonyl, and p-(t-butyl)phenoxycarbonyl), an alkoxycarbonyl group (preferably a substituted or unsubstituted alkoxycarbonyl group having from 2 to 30 carbon atoms, e.g., methoxycarbonyl, ethoxycarbonyl, t-butoxycarbonyl, and n-octadecyloxycarbonyl), a carbamoyl group (preferably a substituted or unsubstituted carbamoyl group having from 1 to 30 carbon atoms, e.g., carbamoyl, N-methylcarbamoyl, N,N-dimethylcarbamoyl, N,N-di-n-octylcarbamoyl, and N-(methylsulfonyl)carbamoyl), an aryl- or heterocyclic azo group (preferably a substituted or unsubstituted arylazo group having from 6 to 30 carbon atoms, a substituted or unsubstituted heterocyclic azo group having from 3 to 30 carbon atoms, e.g., phenylazo, p-chlorophenylazo, and 5-ethylthio-1,3,4-thiadiazol-2-ylazo), an imido group (preferably N-succinimido, N-phthalimido and diacetylamino), a phosphino group (preferably a substituted or unsubstituted phosphino group having from 2 to 30 carbon atoms, e.g., dimethylphosphino, diphenylphosphino and methylphenoxyphosphino), a phosphinyl group (preferably a substituted or unsubstituted phosphinyl group having from 2 to 30 carbon atoms, e.g., phosphinyl, dioctyloxyphosphinyl and diethoxyphosphinyl), a phosphinyloxy group (preferably a substituted or unsubstituted phosphinyloxy group having from 2 to 30 carbon atoms, e.g., diphenoxyphosphinyloxy and dioctyloxyphosphinyloxy), a phosphinylamino group (preferably a substituted or unsubstituted phosphinylamino group having from 2 to 30 carbon atoms, e.g., dimethoxyphosphinylamino and dimethylaminophosphinylamino), and a silyl group (preferably a substituted or unsubstituted silyl group having from 3 to 30 carbon atoms, e.g., trimethylsilyl, t-butyldimethylsilyl and phenyldimethylsilyl).

R preferably represents an unsubstituted alkyl group, an unsubstituted aryl group or an unsubstituted heterocyclic group.

As the alkyl group, e.g., a methyl group, an ethyl group, an n-propyl group, an isopropyl group, a cyclopropyl group, a propargyl group, an n-butyl group, an s-butyl group, an isobutyl group, a t-butyl group, an n-amyl group, a t-amyl group, a cyclopentyl group, an n-hexyl group, a cyclohexyl group, an adamantyl group, an n-octyl group, a t-octyl group, a 2-ethylhexyl group, a dodecyl group, an octadecyl group, a chloromethyl group, a trifluoromethyl group, a methoxymethyl group, a methoxyethoxymethyl group, a methoxythiomethyl group, a tetrahydropyranyl group, a phenacyl group, a cyclopropylmethyl group, an allyl group, a benzyl group, and a trimethylsilylethynyl group are exemplified.

As the aryl group, e.g., a phenyl group, a p-tolyl group, a p-methoxyphenyl group, a naphthyl group, an anthracenyl group, an m-chlorophenyl group, and an o-hexadecanoylaminophenyl group are exemplified.

As the heterocyclic group, e.g., a pyrrolyl group, a pyrrolidinyl group, a thienyl group, a furyl group, a pyridyl group, an imidazolyl group, an indolyl group, a quinolyl group, a pyranyl group, a morpholinyl group, a piperazinyl group, a 2-furyl group, a 2-thienyl group, a 2-pyrimidinyl group, and 2-benzothiazolyl group are exemplified.

X represents a substituent that derives from a nucleophilic agent and is bonded via an oxygen, nitrogen or sulfur atom. X is as described in the nucleophilic agent above.

The compound represented by formula (I) can take the form of a tautomer such as keto type and enol type depending upon the environment. The compound of the present invention is described in only one type of keto type or enol type, but the compound may be the tautomer thereof. The compound represented by formula (II) also may be a tautomer. The compound of the invention is described in one representative form, but tautomers different from the description of the compound of the present invention are also included in the compound of the invention.

The compound represented by formula (II) can be synthesized easily and in a high yield by synthesizing the compound represented by formula (I) by the reaction of the compound represented by formula (III) with malononitrile, and then making a dehydrating agent and then a nucleophilic agent act on the compound represented by formula (I) without being taken out. The concentration of the compound represented by formula (III) at the time of reaction is preferably from 0.01 to 10 mol/liter, and more preferably from 0.1 to 5 mol/liter. It is preferred to use malononitrile in the molar ratio of from 1/10 to 10 to the compound represented by formula (III).

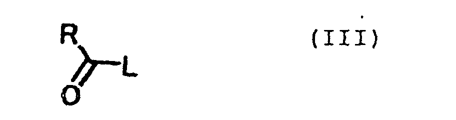

When the compound represented by formula (I) is once taken out, it is necessary to neutralize and precipitate the compound represented by formula (III) and malononitrile with an acid of 1 equivalent amount or more after condensation reaction with a base of 2 equivalent amount or more, which is very uneconomical. Further, there are cases where the yield of crystallization of the compound represented by formula (I) is low, it is also uneconomical to take out the compound represented by formula (I) in this point. Therefore, the synthesizing method of the dicyanomethylidene compound represented by formula (II) comprising the steps of synthesizing the compound represented by formula (I) by reacting the compound represented by formula (III) with malononitrile, and then making a dehydrating agent and then a nucleophilic agent act on the compound represented by formula (I) without taking out the compound represented by formula (I) is excellent.

In formula (III), L represents a releasing group. As the examples of the releasing groups, a halogen atom (e.g., fluorine, chlorine, bromine and iodine), an alkoxyl group, an aryloxy group, an acyloxy group, an alkoxycarbonyloxy group, an aryloxycarbonyloxy group, and a heterocyclic group (e.g., an imidazolyl group) are exemplified. Of the above releasing groups, a halogen atom (in particular, a chlorine atom) is preferred.

The heterocyclic compound represented by the following formula (V) can be easily synthesized by making the compound represented by formula (II) synthesized by the method of the present invention act on hydrazine represented by the following formula (IV). The concentration of the compound represented by formula (II) at the time of reaction is preferably from 0.01 to 10 mol/liter, and more preferably from 0.1 to 5 mol/liter. It is preferred to use hydrazine represented by formula (IV) in the molar ratio of from 1/10 to 10 to the compound represented by formula (II).

**H**_{**2**}**N-NHR'** (IV)

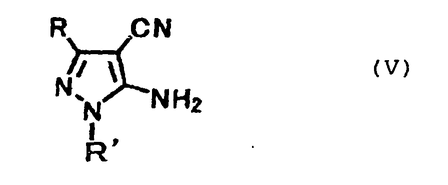

wherein R' represents a hydrogen atom, a substituted or unsubstituted alkyl group, a substituted or unsubstituted aryl group, or a substituted or unsubstituted heterocyclic group. As the substitutedor unsubstitutedalkyl group, the substituted or unsubstituted aryl group, and the substituted or unsubstituted heterocyclic group, the substituents described above in the explanation of R are exemplified.

In the invention, in synthesizing the heterocyclic compound represented by formula (V) by making the compound represented by formula (IV) act on the compound represented by formula (II), this reaction is preferably performed in the presence of a solvent. As the solvent, hydrocarbon-based solvents, e.g., toluene, xylene and hexane, halogen-based solvents, e.g., methylene chloride, sulfoxide-based solvents, e.g., dimethyl sulfoxide, amide-based solvents, e.g., dimethylformamide, ether-based solvents, e.g., tetrahydrofuran, dioxane and diglyme, alcohol-based solvents, e.g., methanol, ethanol and isopropanol, nitrile-based solvents, e.g., acetonitrile, ketone-based solvents, e.g., acetone and cyclohexanone, ester-based solvents, e.g., ethyl acetate, heterocyclic-based solvents, e.g., pyridine and piperidine, and water can be used. Two or more organic solvents may be used as mixture. The solvent may be a hydrous organic solvent or may be a two-phase series solvent of water-organic solvent. Further, an ionic liquid may be used as a reaction solvent as described in J. Chem. Tech. Biotechnol., 68, 351 (1997), or the reaction may be carried out by using a super-critical fluid as described in Science, 269, 1065 (1995).

Of the above solvents, water, alcohol-based solvents, ether-based solvents, nitrile-based solvents, ester-based solvents, heterocyclic-based solvents and mixtures of these solvents are preferably used in the reaction. Water, alcohol-based solvents, e.g., methanol, ethanol and isopropanol, and mixtures of water and alcohol-based solvents are more preferably used.

In the above reaction, it is preferred to carry out the reaction at from 0°C to 200°C, and more preferably from 0°C to 120°C. The termination of the reaction can be confirmed by NMR, TLC, HPLC and other methods.

The time required for the reaction normally depends upon the reaction temperature, the equivalent ratio of the compound represented by formula (IV) to the compound represented by formula (II), and the reaction solvent, but the reaction time is generally about 0.1 to 24 hours.

It is preferred that the reaction is carried out for 0.1 to 24 hours when the temperature is from -10°C to 80°C.

The compounds represented by formulae (IV) and (V) may be accompanied by counter salts by the synthesizing process and the isolation. As the counter salts, e.g., a halogen ion, a sulfate ion, a nitrate ion, a carbonate ion, a sulfonate ion, a phosphate ion, an acetate ion, a metal ion and an ammonium ion are exemplified. The compounds may form inner salts according to the structure.

The compounds for use in the present invention may contain isotopes (e.g., ²H, ³H, ¹³C, ¹⁵N).

### [Example]

The present invention is illustrated in more detail below with reference to examples below.

### EXAMPLE 1

### Synthesis of 2-(1-amino-2,2-dimethylpropylidene)propane-dinitrile

Malononitrile (6.61 g) (0.1 mol) was added to 80 ml of acetonitrile, and 12.3 ml (0.1 mol) of pivaloyl chloride was added thereto. The mixture was cooled to internal temperature of 5°C or lower, and 27.9 ml (0.2 mol) of triethylamine was dropwise added to the mixture with stirring the mixture and with maintaining the internal temperature at 10°C or lower. After completion of the dropwise addition, the reaction mixture was stirred in an ice bath for 30 minutes, and 10.1 ml of phosphorus oxychloride was dropwise added to the reaction mixture so that the internal temperature became 15°C or lower. After stirring the reaction mixture for further two hours in an ice bath, 68 ml of a 25% aqueous ammonia was dropwise added so that the internal temperature lowered to 20°C or less. After stirring the reaction mixture for further one hour in an ice bath, 150 ml of water was dropwise added to the reaction mixture. The solid formed was filtered and washed with water. The solid was dried, thereby 10.47 g (yield: 70%) of the objective 2-(1-amino-2,2-dimethylpropylidene)propanedinitrile was obtained as a white solid. FAB MS (posit) 150 (MH⁺), ¹H NMR (CDCl₃) δ 1.45 (9H, s, t-C₄H₉), 5.78 (1H, bs, NH), 6.35 (1H, bs, NH).

### EXAMPLES 2 TO 11

2-(1-Amino-2,2-dimethylpropylidene)propanedinitrile was synthesized in the same manner as in Example 1 except for using the same mol of dehydrating agents shown below in place of the phosphorus oxychloride. The yield of 2-(1-amino-2,2-dimethylpropylidene)propanedinitrile in each case is shown in Table 1 below.

**TABLE 1**

| Dehydrating Agent | Yield (%) |
|---|---|
| Ms-Cl | 64 |
| Ts-Cl | 43 |
| t-Bu-COCl | 62 |
| Ac-Cl | 32 |
| Ph-COCl | 58 |
| O₂N-C₆H₄-OCOCl | 39 |
| O₂N-C₆H₄-COCl | 41 |
| Ph-OCOCl | 37 |
| Cyanuric chloride | 55 |
| P-Cl₅ | 1 |

### COMPARATIVE EXAMPLE 1

Malononitrile (6.61 g) (0.1 mol) was added to 80 ml of toluene, and 12.3 ml (0.1 mol) of pivaloyl chloride was added thereto. The mixture was cooled to internal temperature of 5°C or lower, and 27.9 ml (0.2 mol) of triethylamine was dropwise added to the mixture with stirring the mixture and with maintaining the internal temperature at 10°C or lower. After completion of the dropwise addition, the reaction mixture was stirred in an ice bath for 30 minutes, and 50 g of ice water was added thereto. To the reaction mixture was dropwise added 13.2 ml of concentrated sulfuric acid so that the internal temperature became 20°C or lower with stirring the reaction mixture in an ice bath. After stirring the reaction mixture for further one hour in an ice bath, the solid formed was filtered and washed with water. The solid was dried, thereby 12.63 g (yield: 84%) of pivaloylmalononitrile was obtained as pale yellow crystals. Pivaloylmalononitrile (15.02 g) (0.1 mol) was added to 80 ml of acetonitrile to lower the internal temperature to 5°C or lower, and 14.0 ml (0.1 mol) of triethylamine was dropwise added with stirring the mixture and with maintaining the internal temperature at 10°C or lower. After completion of the dropwise addition, the reaction mixture was stirred in an ice bath for 30minutes, and 10.1 ml of phosphorus oxychloride was dropwise added to the reaction mixture so that the internal temperature became 15°C or lower. After stirring the reaction mixture for further two hours in an ice bath, 68 ml of a 25% aqueous ammonia was dropwise added to the reaction mixture so that the internal temperature lowered to 20°C or lower. After stirring the reaction mixture for further one hour in an ice bath, 150 ml of water was dropwise added to the reaction mixture. The solid formed was filtered and washed with water. The solid was dried, thereby 9.39 g (yield: 63%) of 2-(1-amino-2,2-dimethylpropylidene)propanedinitrile was obtained as a white solid.

The yield at large of 2-(1-amino-2,2-dimethylpropylidene)propanedinitrile was 53% when pivaloylmalononitrile was once taken out. Therefore, it can be seen that it is also preferred from the point of yield to synthesize 2-(1-amino-2,2-dimethylpropylidene)propanedinitrile by a consistent method without taking out pivaloylmalononitrile.

### EXAMPLE 12

### Synthesis of 5-amino-4-cyano-3-t-butylpyrazole

2-(1-Amino-2,2-dimethylpropylidene)propanedinitrile (7.46 g) (50 mmol) was dispersed in 10 ml of isopropanol and 20 ml of water. After stirring the dispersion at room temperature for about 10 minutes, 7.51 g (150 mmol) of hydrazine hydrate was dropwise added to the dispersion slowly, and the reaction solution was stirred for one hour under reflux with heating.

When the reaction solution was stirred with ice cooling after confirming the completion of the reaction by TLC, crystals were precipitated. The precipitates were filtered, washed with isopropanol and water (4/1), and dried, thereby 7.87 g of the objective compound was obtained as white crystals (yield: 96%). FAB MS (posit) 165 (MH⁺), ¹H NMR (DMSO-d₆) δ 1.27 (9H, s, C₄H₉), 5.50-6.30 (2H, bs, NH₂), 11.20-11.90 (1H, bs, NH).

According to the synthesis method of the present invention, a 3-substituted-5-amino-4-cyanopyrazole compound and a dicyanomethylidene compound which is preferably used as the starting material of 3-substituted-5-amino-4-cyanopyrazole compound can be synthesized in shorter processes and more efficiently than according to synthesizing methods so far been known.

## Claims

1. A synthesizing method of a dicyanomethylidene compound represented by the following formula (II), which comprises: reacting a compound represented by the following formula (I) with a dehydrating agent; and then reacting the reaction product with a nucleophilic agent: 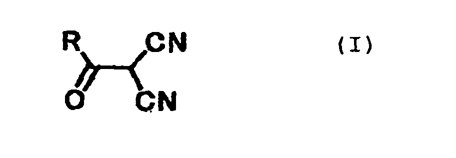 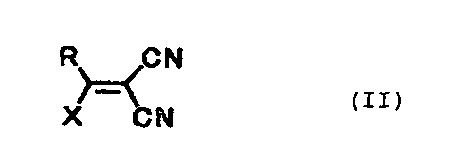 wherein R represents a hydrogen atom, a substituted or unsubstituted alkyl group, a substituted or unsubstituted aryl group, or a substituted or unsubstituted heterocyclic group; X represents a substituent that derives from the nucleophilic agent and is bonded via one of an oxygen atom, a nitrogen atom and a sulfur atom, wherein the dehydrating agent is selected from the group of carboxylic acid halides, chlorocarbonic esters, sulfonic acid chlorides, carbodiimides, isocyanates, carbamic acid chlorides, phosphorus oxychloride, cyanuric chloride, 1,1-carbonyl-bis-1H-imidazole and imidazolinium salts.

2. A synthesizing method of a hetero cyclic compound represented by the following formula (V), which comprises reacting the compound represented by formula (II) synthesized by the method described in claim 1 with a hydrazine compound represented by the following formula (IV):
**H**_{**2**}**N-NHR'** (IV) 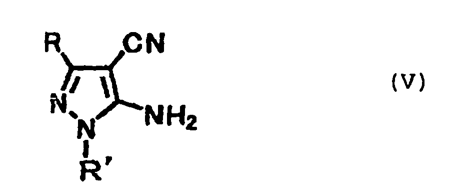
wherein R' represents a hydrogen atom, a substituted or unsubstituted alkyl group, a substituted or unsubstituted aryl group, or a substituted or unsubstituted heterocyclic group.

3. A synthesizing method of a dicyanomethylidene compound represented by the following formula (II), which comprises: synthesizing a compound represented by the following formula (I) by reacting a compound represented by the following formula (III) with malononitrile; reacting the compound represented by the formula (I) with a dehydrating agent without taking out the compound represented by the formula (I); and then reacting the reaction product with a nucleophilic agent: 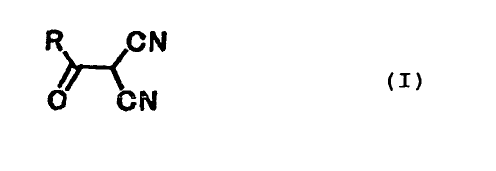 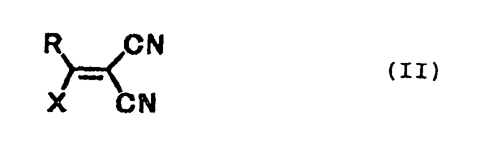 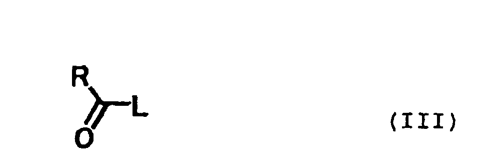 wherein R represents a hydrogen atom, a substituted or unsubstituted alkyl group, a substituted or unsubstituted aryl group, or a substituted or unsubstituted heterocyclic group; X represents a substituent that derives from the nucleophilic agent and is bonded via one of an oxygen atom, a nitrogen atom and a sulfur atom; L represents a releasing group, wherein the dehydrating agent is selected from the group of carboxylic acid halides, chlorocarbonic esters, sulfonic acid chlorides, carbodiimides, isocyanates, carbamic acid chlorides, phosphorus oxychloride, cyanuric chloride, 1,1-carbonyl-bis-1H-imidazole and imidazolinium salts.

4. A synthesizing method of a heterocyclic compound represented by the following formula (V), which comprises reacting the compound represented by formula (II) synthesized by the method described in claim 3 with a hydrazine compound represented by the following formula (IV):
**H**_{**2**}**N-NHR'** (IV) 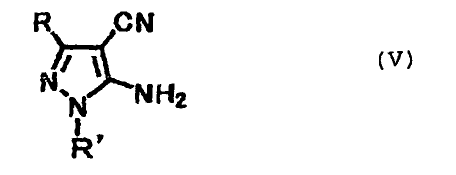
wherein R' represents a hydrogen atom, a substituted or unsubstituted alkyl group, a substituted or unsubstituted aryl group, or a substituted or unsubstituted heterocyclic group.

5. The synthesizing method described in claim 1, wherein the dehydrating agent is used in the molar ratio of from 1/10 to 50 to the compound represented by formula (I).

6. The synthesizing method described in claim 1, wherein the nucleophilic agent is used in the molar ratio of from 1/10 to 50 to the compound represented by formula (I).

7. The synthesizing method described in claim 3 wherein malononitrile is used in the molar ratio of from 1/10 to 10 to the compound represented by formula (III).

8. The synthesizing method described in claim 2 or 4 wherein the hydrazine compound represented by formula (IV) is used in the molar ratio of from 1/10 to 10 to the compound represented by formula (II).

## Patentansprüche

1. Verfahren zur Herstellung einer Dicyanomethylidenverbindung dargestellt durch die folgende Formel (II), umfassend das Reagieren einer Verbindung dargestellt durch die folgende Formel (I) mit einem Dehydriermittel; und anschließend Reagieren des Reaktionsproduktes mit einem nukleophilen Mittel: 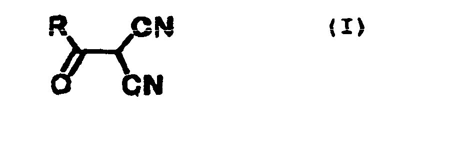 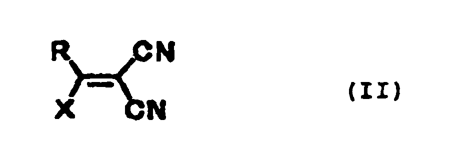 wobei R ein Wasserstoffatom, eine substituierte oder unsubstituierte Alkylgruppe, eine substituierte oder unsubstituierte Arylgruppe, eine substituierte oder unsubstituierte heterozyklische Gruppe darstellt; X einen Substituent darstellt, welcher sich von dem nukleophilen Mittel ableitet und über eines gewählt aus einem Sauerstoffatom, einem Stickstoffatom und einem Schwefelatom gebunden ist, wobei das Dehydriermittel gewählt ist aus der Gruppe der Karbonsäurehalogeniden, Chlorkarbonsäureestern, Sulfonsäurechloriden, Carbodiimiden, Isocyanaten, Carbaminsäurechloriden, Phosphoroxychlorid, Cyanurchlorid, 1,1-Carbonyl-bis-1H-imidazol und Imidazoliniumsalze.

2. Verfahren zur Herstellung einer heterozyklischen Verbindung dargestellt durch die folgende Formel (V), umfassend das Reagieren der Verbindung dargestellt durch die Formel (II), welche durch das in Anspruch 1 beschriebene Verfahren hergestellt ist, mit einer Hydrazinverbindung dargestellt durch die folgende Formel (IV):
**N**_{**2**}**H-NHR'** **(IV)** 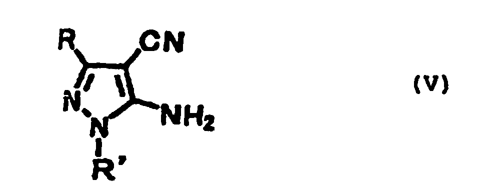
wobei R' ein Wasserstoffatom, eine substituierte oder unsubstituierte Alkylgruppe, eine substituierte oder unsubstituierte Arylgruppe, eine substituierte oder unsubstituierte heterozyklische Gruppe darstellt.

3. Verfahren zur Herstellung einer Dicyanomethylidenverbindung dargestellt durch die folgende Formel (II) umfassend das Herstellen einer Verbindung dargestellt durch die folgende Formel (I) durch das Reagieren einer Verbindung dargestellt durch die folgende Formel (III) mit Malononitril; das Reagieren der Verbindung dargestellt durch die Formel (I) mit einem Dehydriermittel ohne die durch die Formel (I) dargestellte Verbindung herauszunehmen; und anschließend das Reagieren des Reaktionsprodukts mit einem nukleophilen Mittel: 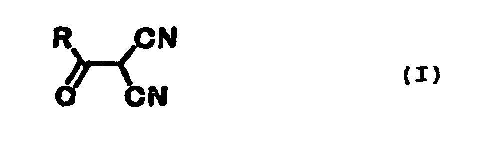 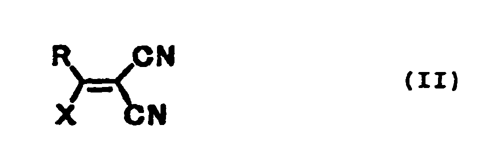 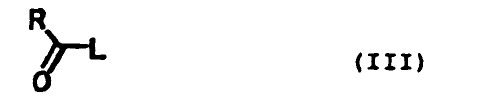 wobei R ein Wasserstoffatom, eine substituierte oder unsubstituierte Alkylgruppe, eine substituierte oder unsubstituierte Arylgruppe, eine substituierte oder unsubstituierte heterozyklische Gruppe darstellt; X einen Substituent darstellt, welcher sich von dem nukleophilen Mittel ableitet und über eins gewählt aus einem Sauerstoffatom, einem Stickstoffatom oder einem Schwefelatom gebunden ist; L eine freisetztende Gruppe darstellt, wobei das Dehydriermittel gewählt ist aus der Gruppe der Karbonsäurehalogeniden, Chlorkarbonsäurestern, Sulfonsäurechloriden, Carbodiimeden, Isocyanaten, Carbaminsäurechloriden, Cyanurchlorid, 1,1-Carbonyl-bis 1H-imindazol und Imidazoliumsalze.

4. Verfahren zur Herstellung einer heterozyklischen Verbindung dargestellt durch die folgende Formel (V), umfassend das Reagieren der Verbindung dargestellt durch die Formel (II), welche durch das in Anspruch 3 beschriebene Verfahren hergestellt wird, mit einer Hydrazinverbindung dargestellt durch die folgende Formel (IV):
**H**_{**2**}**N-NHR' (IV)** 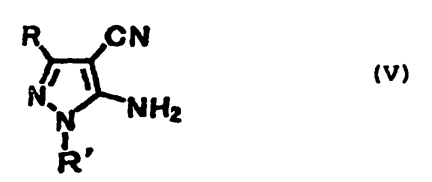
wobei R' ein Wasserstoffatom, eine substituierte oder unsubstituierte Alkylgruppe, eine substituierte oder unsubstituierte Arylgruppe, eine substituierte oder unsubstituierte heterozyklische Gruppe darstellt.

5. Verfahren zur Herstellung nach Anspruch 1, wobei das Dehydriermittel in dem Molarverhältnis von zwischen 1/10 bis 50 zu der Verbindung dargestellt durch die Formel (I) verwendet wird.

6. Syntheseverfahren nach Anspruch 1, wobei das nukleophile Mittel in dem Molarverhältnis von zwischen 1/10 bis 50 zu der Verbindung dargestellt durch die Formel (I) verwendet wird.

7. Syntheseverfahren nach Anspruch 3, wobei Malononitril in dem Molarverhältnis von zwischen 1/10 bis 10 zu der Verbindung dargestellt durch die Formel (III) verwendet wird.

8. Verfahren nach Anspruch 2 oder 4, wobei die Hydrazinverbindung dargestellt durch die Formel (IV) in dem Molarverhältnis von zwischen 1/10 bis 10 zu der Verbindung dargestellt durch die Formel (II) verwendet wird.

## Revendications

1. Procédé de synthèse d'un composé dicyanométhylidène représenté par la formule (II) suivante, lequel comprend : de faire réagir un composé représenté par la formule (I) suivante avec un agent déshydratant ; et ensuite de faire réagir le produit de la réaction avec un agent nucléophile : 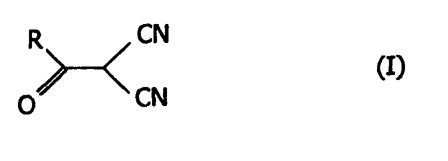 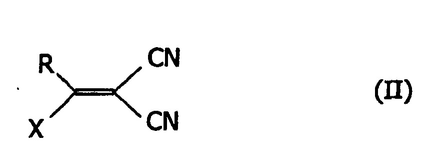 dans lesquelles R représente un atome d'hydrogène, un groupe alkyle substitué ou non substitué, un groupe aryle substitué ou non substitué ou un groupe hétérocyclique substitué ou non substitué ; X représente un substituant qui est dérivé de l'agent nucléophile et qui est lié via un élément parmi un atome d'oxygène, un atome d'azote et un atome de soufre, dans lequel l'agent déshydratant est choisi dans le groupe constitué d'halogénures d'acides carboxyliques, d'esters chlorocarboniques, de chlorures d'acides sulfoniques, de carbodiimides, d'isocyanates, de chlorures d'acides carbamiques, de l'oxychlorure de phosphore, du chlorure cyanurique, du 1,1-carbonyl-bis-1H-imidazole et de sels d'imidazolinium.

2. Procédé de synthèse d'un composé hétérocyclique représenté par la formule (V) suivante, lequel comprend de faire réagir le composé représenté par la formule (II) synthétisé par le procédé décrit dans la revendication 1 avec un composé hydrazine représenté par la formule (IV) suivante :
H₂N ― NHR' (IV) 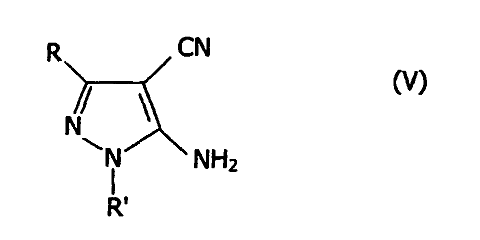
dans lesquelles R' représente un atome d'hydrogène, un groupe alkyle substitué ou non substitué, un groupe aryle substitué ou non substitué ou un groupe hétérocyclique substitué ou non substitué.

3. Procédé de synthèse d'un composé dicyanométhylidène représenté par la formule (II) suivante, lequel comprend : de synthétiser un composé représenté par la formule (I) suivante en faisant réagir un composé représenté par la formule (III) suivante avec un malononitrile ; de faire réagir le composé représenté par la formule (I) avec un agent déshydratant sans enlever le composé représenté par la formule (I) ; et ensuite de faire réagir le produit de la réaction avec un agent nucléophile : 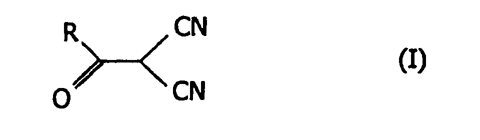 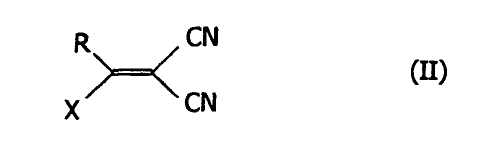 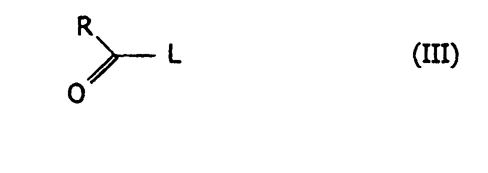 dans lesquelles R représente un atome d'hydrogène, un groupe alkyle substitué ou non substitué, un groupe aryle substitué ou non substitué ou un groupe hétérocyclique substitué ou non substitué ; X représente un substituant qui est dérivé de l'agent nucléophile et qui est lié via un élément parmi un atome d'oxygène, un atome d'azote et un atome de soufre ; L représente un groupe libérable, dans lequel l'agent déshydratant est choisi dans le groupe constitué d'halogénures d'acides carboxyliques, d'esters chlorocarboniques, de chlorures d'acides sulfoniques, de carbodiimides, d'isocyanates, de chlorures d'acides carbamiques, de l'oxychlorure de phosphore, du chlorure cyanurique, du 1,1-carbonyl-bis-1H-imidazole et de sels d'imidazolinium.

4. Procédé de synthèse d'un composé hétérocyclique représenté par la formule (V) suivante, lequel comprend de faire réagir le composé représenté par la formule (II) synthétisé par le procédé décrit dans la revendication 3 avec un composé hydrazine représenté par la formule (IV) suivante :
H₂N ― NHR' (IV) 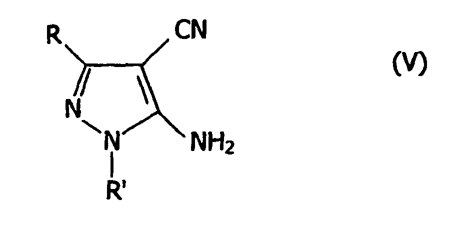
dans lesquelles R' représente un atome d'hydrogène, un groupe alkyle substitué ou non substitué, un groupe aryle substitué ou non substitué ou un groupe hétérocyclique substitué ou non substitué.

5. Procédé de synthèse décrit dans la revendication 1, dans lequel l'agent déshydratant est utilisé avec la proportion molaire allant de 1/10 à 50 par rapport au composé représenté par la formule (I).

6. Procédé de synthèse décrit dans la revendication 1, dans lequel l'agent nucléophile est utilisé avec la proportion molaire allant de 1/10 à 50 par rapport au composé représenté par la formule (I).

7. Procédé de synthèse décrit dans la revendication 3 dans lequel le malononitrile est utilisé avec la proportion molaire allant de 1/10 à 10 par rapport au composé représenté par la formule (III).

8. Procédé de synthèse décrit dans la revendication 2 ou 4 dans lequel le composé hydrazine représenté par la formule (IV) est utilisé avec la proportion molaire allant de 1/10 à 10 par rapport au composé représenté par la formule (II).
